# EUROPEAN PATENT APPLICATION

(11) **EP 1 685 855 A1**
(43) Date of publication of application: **02.08.2006**
(21) Application number: 04818506.0
(22) Date of filing: 12.11.2004
(51) Int. Cl.: A61L 9/01, C09D 7/12, C09D 1/02, C09D 201/00, C09J 11/00, C09J 201/00, D06M 11/71, D06M 23/08

(54) **DEODORANT AND DEODORIZING ARTICLE**

(30) Priority: 14.11.2003 JP 2003385543
(71) Applicant: Idemitsu Technofine Co. Ltd, Sumida-ku, Tokyo 130-0015 (JP)
(72) Inventor: TAGUCHI, Toshiharu, 2990293 (JP); UBARA, Atsuhiko, 2990293 (JP); OYAMA, Shigeru, 2990293 (JP)
(74) Representative: Gille Hrabal Struck Neidlein Prop Roos
(86) International application number: PCT/JP2004/016826
(87) International publication number: WO 2005/046744

(57) **Abstract**

A deodorant includes a powder of an amine salt of a phosphorus inorganic acid, the powder having an average particle diameter of 0.1 to 30 µm. The amine salt of a phosphorus inorganic acid is preferably an ammonium salt. Deodorant products including the deodorant such as an adhesive, a paint and a foamed material can be substantially reduced in formaldehyde emission therefrom, and can deodorize formaldehyde existed in the environment.

## Description

### TECHNICAL FIELD

The invention relates to a deodorant and a deodorant product comprising the deodorant. More particularly, the invention relates to a deodorant having formaldehyde deodorizing capability and products to which the deodorant is applied, specifically, an adhesive, paint, ink, foaming material, fiber treating agent, fiber product, synthetic resin product, building material, paper, and the like.

### BACKGROUND ART

Formaldehyde-containing resins such as a phenol resin, melamine resin, urea-formalin resin, amino alkyd resin, and acrylic resin are used in the field of adhesives, paint, ink, foaming materials, fiber treating agents, fiber products, synthetic resin products, building materials, paper, and the like. These products composed of these resins cause stimulation eyes, headache, and the like due to formaldehyde emission during use. Some people suffer from sick house syndrome accompanied by an ill feeling.
As a countermeasure to the sick house syndrome, the Building Standard Law has recently come into force which obligates installation of a ventilation system and restricts use of plywood.
In addition, there is an increasingly strong desire to have these products themselves had deodorizing capability.

In view of this situation, various products with suppressed formaldehyde emission have been studied. For example, Japanese Patent Applications Laid-open No. 10-237403 and No. 2003-96430 proposed an adhesive composition with suppressed emission of formaldehyde.
Japanese Patent Application Laid-open No. 2003-128982 proposed a method for producing a printing ink with slight formaldehyde emission.
Japanese Patent Application Laid-open No. 2002-187757 proposed a deodorant interior material.

However, these technologies do not necessarily exhibit a sufficient effect of inhibiting formaldehyde emission. Furthermore, there is a desire for a product which uses a material containing no formaldehyde, and can deodorize formaldehyde emission from another product.

The invention has been made in view of the above-described problems and has an object of providing a deodorant possessing an excellent formaldehyde deodorizing capability and a deodorant product which not only inhibits its own formaldehyde emission, but also possesses a function of deodorizing formaldehyde emitted from another product.

### DISCLOSURE OF THE INVENTION

As a result of extensive studies to achieve the above object, the inventors of the invention have found that by incorporating a powder of an amine salt of a phosphorous inorganic acid as a deodorant in the base material forming various products, not only formaldehyde emission from the base material containing formaldehyde can be inhibited, but also a formaldehyde odor in the environment can be deodorized. This finding has led to the completion of the invention.
According to the invention, the following deodorants and deodorant products are provided.
(1) A deodorant comprising a powder of an amine salt of a phosphorous inorganic acid, the powder having an average particle diameter of 0.1 to 30 µm.
(2) The deodorant of (1) above, wherein the amine salt of a phosphorous inorganic acid is an ammonium salt.
(3) A deodorant product comprising the deodorant of (1) or (2).
(4) A deodorant adhesive comprising 1 to 100 parts by mass of the deodorant of (1) or (2) relative to 100 parts by mass of an adhesive.
(5) A deodorant paint comprising 1 to 100 parts by mass of the deodorant of (1) or (2) relative to 100 parts by mass of a paint.
(6) A deodorant foamed material comprising 1 to 100 parts by mass of the deodorant of (1) or (2) relative to 100 parts by mass of a foamable resin.
(7) A deodorant fiber treating agent comprising 1 to 60 parts by mass of the deodorant of (1) or (2).
(8) A fiber product processed by using the deodorant fiber treating agent of (7).
(9) The fiber product of (8), which is a fiber, a woven fabric, or a nonwoven fabric.
(10) A deodorant ink comprising 1 to 100 parts by mass of the deodorant of (1) or (2) relative to 100 parts by mass of a dry ink resin.
(11) A deodorant paper comprising 0.001 to 100 parts by mass of the deodorant of (1) or (2) relative to 100 parts by mass of an absolutely dried pulp.
(12) A deodorant gypsum board comprising a composition comprising 97 to 70 mass% of gypsum and 3 to 30 mass% of the deodorant of (1) or (2).
(13) A deodorant synthetic resin product comprising 1 to 100 parts by mass of the deodorant of (1) or (2) relative to 100 parts by mass of a synthetic resin.
(14) A plywood board comprising the deodorant of (1) or (2) applied thereon.
(15) A hot melt product comprising 1 to 30 parts by mass of the deodorant of (1) or (2) relative to 100 parts by mass of a hot melt agent.

A deodorant having excellent formaldehyde deodorization capability can be provided by the invention. A deodorant product that not only can inhibit formaldehyde emission therefrom, but also can deodorize formaldehyde emission from another product can be provided by adding the deodorant.

### BEST MODE FOR CARRYING OUT THE INVENTION

The deodorant and deodorant product of the invention will now be specifically described.
The deodorant contains an amine salt of a phosphorous inorganic acid with an average particle diameter of 0.1 to 30 µm. The average particle diameter is the particle size measured using a laser diffraction particle size distribution analyzer.
As the amine salt of a phosphorous inorganic acid used in the invention, amine salts of a phosphorous inorganic acid such as phosphoric acid, phosphorous acid, pyrophosphoric acid, polyphosphoric acid, hypophosphorous acid, or metaphosphoric acid can be given.
Examples of the amine compound that forms an amine salt with the phosphorous inorganic acid include ammonia, phenylhydrazine, hydrazylphenol, urea, thiourea, semicarbazide, carbazone, 1,5-diphenylcarbanohydrazide, thiocarbazone, ethylenediamine, hexamethylenetriamine melamine, cyclohexanediamine, naphthalenediamine, aniline, tetramethylenediamine, 1,2,5-pentanetriamine, 2-amino-1,3,5-triazine, triethylamine, triethanolamine, 1-aminopiperazine, acetamidine, benzamidrazone, 3,5-diphenylformazone, carbodiimide, guanidine, 1,1,3-trimethylguanidine, 3,4-dimethyl-iso-semicarbazide, thiocarbazone, and thiocarbodiazone.
As the amine compound, amino acids possessing amino groups such as lysine, arginine, ornithine, and proline can be preferably used.

Of these amine salts of a phosphorous inorganic acid, ammonium salts of a phosphorous inorganic acid are preferable from the viewpoint of safety. Ammonium salts such as ammonium phosphate, ammonium dihydrogen phosphate, diammonium hydrogen phosphate, ammonium polyphosphate, ammonium cyclohexaphosphate and the like are particularly preferable due to excellent heat stability, excellent deodorant performance, and impartment of flame-retardant properties. Of these ammonium salts, ammonium polyphosphate, particularly ammonium polyphosphate containing water insoluble matter of 85% or more, preferably 90% or more, at 20°C is preferable due to low water solubility at room temperature and for securing durability of the products for uses other than a plywood.
Commercially available products of the amine salt of phosphorous inorganic acid may be used.

An average particle diameter of the powdery amine salt of phosphorous inorganic acid is from 0.1 to 30 µm. An average particle diameter of less than 0.1 µm is unsuitable due to low deodorant performance and necessity of prevention of powder scattering in the process steps.
On the other hand, if the average particle diameter is more than 30 µm, the surface area of the amine salt becomes small so that the performance is hardly exhibited. In addition, a problem of a coarse surface occurs according to the product to which the deodorant is added. Mentioning specific products, in the case of an adhesive, the coarse surface decreases adhesiveness, and in the case of a paint, paper, or fiber treating agent, the coarse surface impairs external appearances of the painted products, printed matter, and fiber products.
The average particle diameter of the amine salt powder is preferably from 3 to 30 µm, and particularly preferably from 5 to 20 µm. If less than 3 µm, not only particles easily agglomerate, leading to difficult handling, but also particle size reduction of the amine salt is difficult.
In addition to the amine salt of a phosphorous inorganic acid, inorganic compounds such as porous silica, activated carbon, zeolite, activated clay, silica gel, alumina, montmorillonite, titanium oxide, zinc oxide, and iron oxide and amine compounds such as organic silicon amines, aromatic amines, and hydrazine derivatives can be added to the deodorant of the invention.

Due to inclusion of the amine salt powder of a phosphorous inorganic acid, the deodorant of the invention exhibits excellent capability of deodorizing formaldehyde. Therefore, if added to various products in which a basic component containing formaldehyde is used, such as adhesives and building materials, the deodorant of the invention can significantly inhibit formaldehyde emission from the basic component. In addition to inhibition of formaldehyde emission from those products, the deodorant can deodorize formaldehyde emission from other products.
Examples of the deodorant products in which the deodorant of the invention is used will now be described. The deodorant product of the invention, however, is not limited to the examples described below.

### <Deodorant adhesive>

The deodorant adhesive of the invention contains an adhesive resin and the deodorant.
As examples of the adhesive resin, a polyvinyl-alcohol resin, urea resin, phenol resin, melamine resin, isocyanate resin, polyester resin, acrylic resin, urethane epoxy resin, rubber-based resin, ethylene-vinyl acetate copolymer resin, polyvinyl-acetate resin, and acrylic-ester polymer can be given.
The deodorant of the invention can be particularly suitably used together with a methylol group-containing urea resin, phenol resin, melamine resin, and acrylic resin materials containing formaldehyde.
These resins and the above-mentioned deodorant are blended with water or a solvent to prepare the deodorant adhesive.
The adhesive can be prepared according to a conventional method using commonly used solvents and additives. For example, the methods described in Japanese Patent Applications Laid-open No. 10-237403 and No. 2003-96430 can be referred to.
The deodorant adhesive of the invention can also be prepared by adding the above deodorant to a commercially available adhesive.

The amount of the above amine salt deodorant of phosphorous inorganic acid to be added to 100 parts by mass of the adhesive (which indicates all components excepting the deodorant of the invention) is from 1 to 100 parts by mass, preferably 3 to 40 parts by mass, and particularly preferably 5 to 30 parts by mass. If the amount is less than 1 part by mass, the deodorization and antibacterial effect cannot be expected. If the amount exceeds 100 parts by mass, the adhesiveness decreases due to an excessive amount of the deodorant. An excessive addition of the deodorant is also undesirable in view of economy.

### <Deodorant paint>

The deodorant paint of the invention contains a paint resin and the deodorant.
As the paint resin, an acrylic resin, polyurethane resin, fluororesin, silicone resin, acrylic-styrene resin, styrene resin, vinyl-chloride resin, vinyl-acetate resin, vinyl-acetal resin, polyester resin, amino resin, epoxy resin, and the like can be given. Particularly suitably used are materials in which a methylol group-containing epoxy resin, acrylic resin, and formaldehyde condensate are added.
These resins and the above-mentioned deodorant are blended with water or a solvent to prepare the deodorant paint.
The paint can be prepared according to a conventional method using commonly used solvents and additives in the field of paint. For example, the method described in Japanese Patent Application Laid-open No. 2002-322424 can be referred to.
The deodorant paint of the invention can also be prepared by adding the above deodorant to a commercially available paint.
The deodorant paint can be applied to a plywood, paper, metal plate, plastic plate, and the like.
The amount of the above amine salt deodorant of phosphorous inorganic acid to be added to 100 parts by mass of the paint (which indicates all components excepting the deodorant of the invention) is the same as that mentioned above for the deodorant adhesive.

### <Deodorant foamed material>

Various common resins such as a urethane resin, polystyrene, polypropylene, and polyethylene can be utilized as the foamable resin used for producing the foamed material. Particularly suitably used are urethane resin materials in which a methylol group-containing formaldehyde condensate is added.
The deodorant foamed material can be prepared by mixing these resins or resin materials with the above deodorant and foaming the mixture.
The foamed material can be prepared according to a conventional method using various commonly-used additives in the field of foamed product. For example, Japanese Patent Application Laid-open No. 8-269157 can be referred to for the production of urethane foam.
The amount of the above amine salt deodorant of phosphorous inorganic acid to be added to 100 parts by mass of the foamable resin is the same as that mentioned above for the deodorant adhesive.

### <Deodorant fiber treating agent>

As the basic component for producing a fiber treating agent, aqueous emulsions of urethane resin, acrylic resin, and their copolymers can be given. Materials containing an acrylic resin having a methylol group and its copolymer are particularly preferable.
The deodorant fiber treating agent can be prepared by mixing these resins with the above deodorant.
The fiber treating agent can be prepared according to a conventional method. Various additives commonly used in the field of fiber treating agents can be used.

The amount of the amine salt deodorant of phosphorous inorganic acid added to the deodorant fiber treating agent is from 1 to 60 mass%, preferably from 3 to 50 mass%, and particularly preferably from 5 to 30 mass%.
In addition to aqueous emulsion processing, a deodorization treatment is also possible by dust collecting processing using fine particles with a diameter of 1 µm or less in the fiber product dyeing step.
Fiber products such as a fiber, woven fabric, and nonwoven fabric which are processed using this deodorant fiber treating agent exhibit excellent capability of deodorizing formaldehyde.

### <Deodorant ink>

As examples of the basic component used for producing ink, an acrylic resin, styrene resin, vinyltoluene resin, rosin ester resin, rubber emulsion, ethylene-vinyl-acetate copolymer emulsion, vinyl-chloride resin, vinyl-acetate resin, aqueous emulsion such as polyvinyl-acetate emulsion or an acrylic-ester polymer emulsion, rosin-modified phenol resin, alkyd resin, urethane-acrylate resin, epoxy acrylate resin, polyester acrylate resin, and unsaturated-polyester resin can be given.
A methylol group-containing rosin-modified phenol resin, epoxy acrylate resin, alkyd resin, and acrylic resin are particularly preferably used.
The deodorant ink can be prepared by mixing these resins with the above deodorant.
The ink can be prepared according to a conventional method. Various additives commonly used in the field of inks can be used. For example, the method described in Japanese Patent Application Laid-open No. 2001-164169 can be referred to.
The deodorant ink of the invention can also be prepared by adding the above deodorant to a commercially available ink.
The amount of the above amine salt deodorant of phosphorous inorganic acid to be added to 100 parts by mass of the dry ink resin, which is a product prepared by drying the above resin, is the same as that mentioned above for the deodorant adhesive.

### <Deodorant paper>

In the paper-making process for newspaper or phone directories, in addition to polyethylene oxide, a phenol resin is used for increasing the yield and filter performance (for example, refer to Japanese Patent Application Laid-open No. 9-188993). For this reason, a problem of formaldehyde emission from the paper products occurs.
According to the invention, the formaldehyde emission can be prevented by adding 0.001 to 20 mass%, preferably 0.002 to 15 mass%, and particularly preferably 0.003 to 10 mass% of the above amine salt powder of phosphorous inorganic acid to an absolutely dried pulp used as the basic component.
The paper can be prepared according to a conventional method. For example, the method described in Japanese Patent Application Laid-open No. 9-188993 can be referred to.

### <Deodorant gypsum board>

The deodorant gypsum board of the invention contains a composition comprising gypsum and the deodorant of the invention. The gypsum board includes, in addition to the gypsum boards used as a common interior building material, a gypsum board with holes for sound absorption, a wood-wool gypsum board, and a glass-fiber reinforced gypsum board.
As the gypsum, half-hydrate gypsum produced by sintering natural gypsum or chemical gypsum can be used as a main material. A gypsum board can be produced by adding water to the half-hydrate gypsum, followed by mixing, forming, and hardening. Occasionally, dihydrate gypsum before sintering is used.
The above deodorant of amine salt powder of phosphorous inorganic acid is added in the process for producing the gypsum board to obtain the deodorant gypsum board. The deodorant may be added to the gypsum in the state of aqueous slurry or may be added in the state of powder when the gypsum is mixed with water.
Various additives commonly used in gypsum boards can be added to the deodorant gypsum board of the invention. For example, the method described in Japanese Patent Application Laid-open No. 2002-187757 can be referred to.

The amount of the deodorant to be added to the gypsum is 3 to 30 mass%, preferably 5 to 15 mass%, and particularly preferably 5 to 10 mass%. If the amount is less than 3 mass%, a deodorant effect cannot be expected; and if the amount exceeds 30 mass%, the amount is too large and it is undesirable in view of properties and economy.
The deodorant gypsum board of the invention has thermal insulation properties, gas permeability, and an effect of deodorizing formaldehyde emitted from plywood and furniture.
The deodorant of the invention can be applied not only to gypsum boards, but also to cement boards used as an interior building material.

### <Deodorant synthetic resin product>

As synthetic resin for producing the deodorant synthetic resin product, various resins such as various general-purpose resins such as polystyrene, polypropylene, and polyethylene, acrylic resin, polyurethane resin, fluororesin, vinyl-chloride resin, vinyl-acetate resin, nylon resin, polyester resin, polycarbonate resin, and the like can be given.
The deodorant synthetic resin product can be obtained by adding the above amine salt powder of phosphorous inorganic acid to these synthetic resins according to required characteristics, and forming the mixture into an injection formed product, film, nonwoven fabric, hollow formed product, heat formed product, or the like by conventional forming method such as extrusion molding, injection molding, or the like.

The amount of the above amine salt deodorant of phosphorous inorganic acid to be added to 100 parts by mass of the synthetic resin is the same as that mentioned above for the deodorant adhesive.
Various commonly-used additives may be added to the deodorant synthetic resin product of the invention.
The deodorant may be suitably used in materials to which a methylol group-containing formaldehyde condensate is added.
The deodorant synthetic resin product of the invention can be applied to vehicle material formed products such as an instrumental panel and door trim, an outer covering material and resin wallpaper, films such as ground waterproofing film in buildings, and the like.

As the application for preventing formaldehyde emission from products, a plywood for the interior of building materials and the like and a hotmelthot melt agent for application to vehicles and the like can be given.
There are legal restrictions to the application in this field. A formaldehyde emission inhibitor having high safety used for these applications is extremely useful in the field of industry.
Conventionally, hydrazide compounds have been mainly used in these applications, and it has been seen that they had a problem with safety.

### <Plywood>

The plywood of the invention is made by applying the above deodorant to a plywood.
When the deodorant of the invention is applied to a plywood, the deodorant is dissolved in an aqueous solution, a paint or the like, or emulsified with a surfactant into an aqueous emulsion, followed by simple application to a plywood by spraying or the like, thereby formaldehyde emission can be effectively prevented. The deodorant is added to the coating solution in an amount of about 0.5 to 20 mass% to prepare a solution, paint, or aqueous emulsion to be applied to a plywood by spraying. The amount to be applied is appropriately adjusted according to the amount of formaldehyde emitted from the plywood.
Application in an amount of 1 to 5 g/m² on a dry solid basis is preferable.
Among the deodorants of the invention, ammonium phosphate, ammonium dihydrogen phosphate, or diammonium hydrogen phosphate can be preferably used due to the water-solubility thereof. Of these, ammonium phosphate with a 40% (at 20°C) or more solubility in water, and diammonium hydrogen phosphate are particularly preferable.

### <Hot melt agent>

The hot melt agent of the invention is used by adding the above deodorant, or the above deodorant in combination with a general deodorant to a common hot melt agent.
The hot melt agent of the invention is useful as a vehicle interior material, interior boards of buildings, and the like, particularly in vehicle application. In the vehicle application, the hot melt agent is suitably used for heat adhesion of interior plastic boards used as a ceiling material with interior non-woven fabric or interior cloth inside a car.
As examples of the hot melt agent, an olefin-based hot melt agent, polyurethane hot melt agent, ethylene-vinyl acetate resin copolymer hot melt agent, styrene copolymer hot melt agent, nylon hot melt agent, and polyester hot melt agent can be given. In this application, the hot melt agent is used as a heat adhesive sheet for causing an interior board to adhere to an interior cloth, interior paper, or interior nonwoven fabric to reduce formaldehyde concentration. The deodorant effect can be improved by subjecting the heat adhesive sheet of the deodorant hot melt agent composition to a drawing process.
The amount of the above amine salt of phosphorous inorganic acid to be added to 100 parts by mass of the hot melt agent is 1 to 30 mass%, and preferably 5 to 20 mass%.

### EXAMPLES

The invention will now be described more specifically by way of examples.

### Example 1

A deodorant adhesive was prepared by adding 10 parts by mass of ammonium dihydrogen phosphate (manufactured by Wako Pure Chemical Industries, Ltd.), with an average particle diameter of 5 µm, to 100 parts by mass of an acrylic resin adhesive ("Y650" a mixture of A agent and B agent, manufactured by Cemedine Co., Ltd.) and mixing the two ingredients.
The average particle diameter of the ammonium dihydrogen phosphate was measured using a laser diffraction particle size distribution analyzer ("LMS-24" manufactured by Seishin Enterprise Co., Ltd.).
The formaldehyde emission from the adhesive was evaluated by the following method.
A measuring sample was prepared by applying a sheet of A4 size regular paper to a plywood board with a thickness of 10 mm using 5 ml of the adhesive. Test specimens with a size of 4 × 4 cm were cut out from the sample. One piece of the test specimens was put into a 500 ml broad-mouth bottle and the bottle was covered.
The bottle was heated at 60°C for 5 minutes and allowed to stand. Then, the formaldehyde concentration in the bottle was measured using a Kitagawa gas detector tube.
As a result, the formaldehyde concentration in the bottle was found to be 0.5 ppm or less.
The composition of the adhesives and the results of the formaldehyde emission test in the Example 1 and the following Example 2 and Comparative Examples 1 to 3 are shown in Table 1.

**TABLE 1**

| | Component | Example | | Comparative Example | | |
|---|---|---|---|---|---|---|
| | | 1 | 2 | 1 | 2 | 3 |
| Blend ratio (part by mass) | Acrylic resin adhesive | 100 | 100 | 100 | 100 | 100 |
| | Ammonium dihydrogen phosphate *1 | 10 | 6 | 0 | 0 | 5 |
| | Sodium dihydrogen phosptiate | - | - | - | 5 | - |
| Formaldehyde emission test (ppm) | | 0.5 or less | 0.5 or less | 2 | 3 | 2 |

| | | | | | | |
|---|---|---|---|---|---|---|
| *1: The particle diameter of ammonium dihydrogen phosphate used in Examples 1 and 2 was 5 µm and that used in Comparative Example 3 was 34 µm. | | | | | | |

### Example 2

An adhesive was prepared and evaluated in the same manner as in Example 1 except that the ammonium dihydrogen phosphate was added in an amount of 6 parts by mass.
As a result, the formaldehyde concentration in the bottle was found to be 0.5 ppm or less.

### Comparative Example 1

Formaldehyde emission from an adhesive to which in Example 1, no ammonium dihydrogen phosphate was added, was measured in the same manner as in Example 1. As a result, the formaldehyde concentration in the bottle was found to be 2 ppm.

### Comparative Example 2

An adhesive was prepared and evaluated in the same manner as in Example 1 except for adding 5 parts by mass of sodium dihydrogen phosphate powder with an average diameter of 5 µm (manufactured by Wako Pure Chemical Industries, Ltd.) instead of ammonium dihydrogen phosphate.
As a result, the formaldehyde concentration in the bottle was found to be 3 ppm.

### Comparative Example 3

An adhesive was prepared and evaluated in the same manner as in Example 1 except for adding 5 parts by mass of classified ammonium dihydrogen phosphate with an average particle diameter of 34 µm instead of ammonium dihydrogen phosphate with an average particle diameter of 5 µm.
As a result, the formaldehyde concentration in the bottle was found to be 2 ppm.

### Example 3

A paint was prepared by adding 30 parts by mass of the same ammonium dihydrogen phosphate with an average particle diameter of 5 µm as used in Example 1 to 100 parts by mass of an acrylic resin paint ("Aqueous Fresh 21" manufactured by Dai Nippon Toryo Co., Ltd.) and mixing the ingredients.
The formaldehyde emission of this paint was evaluated in the same manner as in Example 1 using a measuring sample prepared by spraying 100ml of the paint onto a A4 size plywood board with a thickness of 10 mm.
As a result, the formaldehyde concentration in the bottle was found to be 0.5 ppm or less.
The composition of the paints and the results of the formaldehyde emission test in the Example 3 and the following Example 4 and Comparative Examples 4 and 5 are shown in Table 2.

**TABLE 2**

| | Component | Example | | Comparative Example | |
|---|---|---|---|---|---|
| | | 3 | 4 | 4 | 5 |
| Blend ratio (part by mass) | Acrylic resin adhesive | 100 | 100 | 100 | 100 |
| | Ammonium dihydrogen phosphate | 30 | 20 | 0 | 0 |
| | Sodium dihydrogen phosphate | - | - | - | 5 |
| Formaldehyde emission test (ppm) | | 0.5 or less | 0.5 or less | 4 | 3 |

### Example 4

A paint was prepared and evaluated in the same manner as in Example 3 except for adding 20 parts by mass of the ammonium dihydrogen phosphate.
As a result, the formaldehyde concentration in the bottle was found to be 0.5 ppm or less.

### Comparative Example 4

Formaldehyde emission from a paint to which in Example 3, no ammonium dihydrogen phosphate was added, was measured in the same manner as in Example 1. As a result, the formaldehyde concentration in the bottle was found to be 4 ppm.

### Comparative Example 5

A paint was prepared and evaluated in the same manner as in Example 3 except for adding 5 parts by mass of the same sodium dihydrogen phosphate powder as used in Comparative Example 2 instead of ammonium dihydrogen phosphate.
As a result, the formaldehyde concentration in the bottle was found to be 3 ppm.

### Example 5

An ink was prepared by adding 5 parts by mass of the same ammonium dihydrogen phosphate as used in Example 1 to 100 parts by mass of a rosin phenol resin ink (manufactured by Dainippon Ink Co., Ltd., resin content: 23 weight%) and mixing the ingredients.
The formaldehyde emission of this ink was evaluated in the same manner as in Example 1 using a measuring sample prepared by applying 100 ml of the ink to a A4 size regular paper.
As a result, the formaldehyde concentration in the bottle was found to be 0.5 ppm or less.
The amount of ammonium dihydrogen phosphate added and the results of the formaldehyde emission test using the inks prepared in the Example 5 and the following Comparative Example 6 are shown in Table 3.

**TABLE 3**

| | Example 5 | Comparative Example 6 |
|---|---|---|
| Amount of ammonium dihydrogen phosphate (part by mass) | 5 | 0 |
| Formaldehyde emission test (ppm) | 0.5 or less | 4 |

### Comparative Example 6

Formaldehyde emission from an ink to which in Example 5, no ammonium dihydrogen phosphate was added, was measured in the same manner as in Example 1. As a result, the formaldehyde concentration in the bottle was found to be 4 ppm.

### Example 6

An urethane foam was prepared by mixing 100 parts by mass of polyol ("#3000" manufactured by Dow Polyurethane Co., Ltd.), 40 parts by mass of isocyanate ("T-80" manufactured by Dow Polyurethane Co., Ltd.), 3 parts by mass of water, 0.3 part by mass of an amine-based catalyst ("33LV" manufactured by Air Products and Chemicals, Inc.), 0.3 part by mass of an amine-based catalyst ("AT33" manufactured by Air Products and Chemicals, Inc.), 0.3 part by mass of a tin catalyst ("T-9" manufactured by Nitto Kasei Co., Ltd.), 3 parts by mass of a foam adjusting agent (a product of Nippon Unicar), 8 parts by mass of the same ammonium dihydrogen phosphate as used in Example 1, and 3 parts by mass of melamine formaldehyde condensate salt.
Formaldehyde emission was measured in the same manner as in Example 1 using a test specimen of a 5 cm × 5 cm square piece cut from this urethane foam.
As a result, the formaldehyde concentration in the bottle was found to be 0.5 ppm or less.
The amount of ammonium dihydrogen phosphate added and the results of the formaldehyde emission test using the urethane foam prepared in Example 6 and the following Comparative Example 7 are shown in Table 4.

**TABLE 4**

| | Example 6 | Comparative Example 7 |
|---|---|---|
| Amount of ammonium dihydrogen phosphate (part by mass) | 8 | 0 |
| Formaldehyde emission test (ppm) | 0.5 or less | 5 |

### Comparative Example 7

An urethane foam was prepared and evaluated in the same manner as in Example 6 except that no ammonium dihydrogen phosphate was added. As a result, the formaldehyde concentration in the bottle was found to be 5 ppm.

### Example 7

A deodorant fiber treating agent (containing 25 mass% of ammonium dihydrogen phosphate) was prepared by adding 100 parts by mass of a 50 mass% aqueous emulsion of the same ammonium dihydrogen phosphate as used in Example 1 to 100 parts by mass (on dry basis) of an acrylic emulsion of a fiber treating agent ("T-15" manufactured by Ganz Chemical Co., Ltd., aqueous emulsion with a resin content of 50 mass%).
This deodorant fiber treating agent was applied to A4 size polyester ground fabric using a bar coater in an amount of 100 g/m² to prepare a measuring sample. Formaldehyde emission was measured in the same manner as in Example 1 using a test specimen of a 5 cm × 5 cm square piece cut from this ground fabric.
As a result, the formaldehyde concentration in the bottle was found to be 0.5 ppm or less.
The amount of ammonium dihydrogen phosphate added and the results of the formaldehyde emission test using the fiber treating agent prepared in the Example 7 and the following Comparative Example 8 are shown in Table 5.

**TABLE 5**

| | Example 7 | Comparative Example 8 |
|---|---|---|
| Amount of ammonium dihydrogen phosphate (part by mass) | 25 | 0 |
| Formaldehyde emission test (ppm) | 0.5 or less | 10 |

### Comparative Example 8

A fiber treating agent was prepared and evaluated in the same manner as in Example 7 except that no ammonium dihydrogen phosphate was used. As a result, the formaldehyde concentration in the bottle was found to be 10 ppm.

### Example 8

A deodorant gypsum board with a thickness of 10 mm, a width of 910 mm, and a specific gravity of 0.7 was prepared from 10 parts by mass of the same ammonium dihydrogen phosphate as used in Example 1 to 100 parts by mass of gypsum according to the method described in Japanese Patent Application Laid-open No. 2002-187757.
The formaldehyde emission from the gypsum board was evaluated by the following method.
A 4 cm × 4 cm square specimen was cut from the gypsum board and put into a 500 ml broad-mouth bottle. 2 µml of 1% formaldehyde aqueous solution was introduced into the broad-mouth bottle using a micro-syringe. The bottle was covered and sealed. After allowing the bottle to stand at room temperature for two hours, the formaldehyde concentration in the bottle was measured.
As a result, the formaldehyde concentration in the bottle was found to have been reduced to 0.5 ppm or less.
The amount of ammonium dihydrogen phosphate added and the results of the formaldehyde emission test using the gypsum boards prepared in the Example 8 and the following Comparative Example 9 are shown in Table 6.

**TABLE 6**

| | Example 8 | Comparative Example 9 |
|---|---|---|
| Amount of ammonium dihydrogen phosphate (part by mass) | 10 | 0 |
| Formaldehyde emission test (ppm) | 0.5 or less | 20 |

### Comparative Example 9

A gypsum board was prepared and evaluated in the same manner as in Example 8 except that no ammonium dihydrogen phosphate was added. As a result, the formaldehyde concentration was found to be 20 ppm or less.

### Example 9

A paper milling raw slurry was prepared by adding 10 parts by mass of talc, 1 part by mass of a cationized starch, 0. 3 part by mass of a sizing agent, 0.3 part by mass of a phenolic sizing auxiliary agent, 0.01 part by mass of an yielding agent, and 0. 05 part by mass of the same ammonium dihydrogen phosphate as used in Example 1 to 100 parts by mass of an aqueous pulp slurry (absolutely dried pulp content: 1 mass%) made from a 8:2 (mass ratio) mixture of broadleaf tree pulp and needle-leaf tree pulp.
This slurry was formed into paper with a density of 65 g/m² using a sheeting machine. The paper was press-dehydrated and dried at 100°C for 80 seconds to obtain a deodorant paper.
Formaldehyde emission was measured in the same manner as in Example 1 using a test specimen of a 5 cm × 5 cm square piece cut from this paper.
As a result, the formaldehyde concentration in the bottle was found to be 0.8 ppm.
The amount of ammonium dihydrogen phosphate added and the results of the formaldehyde emission test using the papers prepared in the Example 9 and the following Comparative Example 10 are shown in Table 7.

**TABLE 7**

| | Example 9 | Comparative Example 10 |
|---|---|---|
| Amount of ammonium dihydrogen phosphate (part by mass) | 0.05 | 0 |
| Formaldehyde emission test (ppm) | 0.8 | 3 |

### Comparative Example 10

A paper was prepared and evaluated in the same manner as in Example 9 except that no ammonium dihydrogen phosphate was added. As a result, the formaldehyde concentration in the bottle was found to be 3 ppm.

### Example 10

A high pressure dying machine was charged with an undyed polyester cloth and a 3 mass% aqueous emulsion of ammonium dihydrogen phosphate with an average particle diameter of 0.2 µm (manufactured by Wako Pure Chemical Industries, Ltd.), followed by addition of palanil brilliant blue BGF dye (manufactured by BASF) in an amount adjusted to 1 mass%. The mixture was heated over a hot bath at 120°C for 60 minutes to prepare a polyester cloth. A deodorant cloth containing 1 mass% of ammonium dihydrogen phosphate relative to the weight of the polyester cloth was obtained by reduction cleaning, air drying, staining, and deodorization treatment according to the method described in Japanese Patent Publication No. 5-12475.
Formaldehyde deodorizing capability was evaluated in the same manner as in Example 8 using a 4 cm × 4 cm square piece cut from this cloth. As a result, the formaldehyde concentration in the bottle was found to have been reduced to 0.5 ppm or less.
The formaldehyde concentration was 21 ppm in a control test carried out without placing the deodorant cloth in the bottle.

### Example 11

90 mass% of polypropylene ("Idemitsu PP F740N" manufactured by Idemitsu Petrochemical Co., Ltd.) and 10 mass% of ammonium dihydrogen phosphate with an average particle diameter of 24 µm in the total amount of 20 kg were dry-blended. The blend was kneaded using an extruder (manufactured by Ikegai Corp., diameter: 50 mm) at 240°C to produce a polypropylene resin composition.
The composition was processed into a deodorant polypropylene sheet with a width of 30 cm and a thickness of 200 µm at 230°C using a 50 mm sheet forming machine.
Formaldehyde deodorizing capability was evaluated in the same manner as in Example 8 using a 4 × 4 cm square piece cut from this deodorant sheet. As a result, the formaldehyde concentration in the bottle was found to have been reduced to 3 ppm.
The formaldehyde concentration was 22 ppm in a control test carried out without placing the deodorant sheet in the bottle.

In addition, the sheets were bonded together using a formaldehyde-containing adhesive and cut into a 4 cm × 4 cm square piece, and the piece was put into a 500 ml broad-mouth bottle. After heating the bottle in an oven at 80°C for 24 hours, the formaldehyde concentration in the bottle was measured using a Kitagawa gas detector tube to find that the concentration was 0.5 ppm or less.
In a test using a blank PP instead of this sheet, the formaldehyde concentration in the bottle was 1 ppm.

### Example 12

A 10% aqueous solution of ammonium phosphate with an average particle diameter of 20 µm (manufactured by Wako Pure Chemical Industries, Ltd.) was prepared and applied to a plywood board with a dimension of 15 cm × 50 cm × 3 mm using a simple spray in an amount adjusted to be 25 g/m² (wet weight), thereby providing a formaldehyde-emission proofing treatment.
The plywood board was allowed to stand for three days after the treatment. Then, each of the treated and untreated plywood boards was cut into ten 15 cm × 5 cm pieces. According to the formaldehyde emission amount measuring method for normal plywood of the Japanese Agricultural Standards, formaldehyde emitted from the plywood boards was absorbed in distilled water for colorimetrical analysis of the concentration of formaldehyde in the absorbed water by the acetyl acetone method using a spectrophotometer. The concentration of the treated board was 0.5 mg/l, whereas the concentration of the untreated board was 0.8 mg/l.

### Example 13

Ammonium polyphosphate with an average particle diameter of 20 µm (manufactured by Taihei Chemical Industrial Co. , Ltd.) was added to ethylene-vinyl acetate copolymer resin (content of vinyl acetate: 30 mass%) in an amount of 10 mass%. The mixture was kneaded at 130°C in a roll kneader to obtain a 20 cm × 20 cm sheet with a thickness of 200 µm.
The sheet was heat-laminated with a polyester nonwoven fabric with a density of 150 g/m² using a press forming machine at 130°C for two minutes to obtain a nonwoven fabric with a deodorant hot melt agent.
This deodorant nonwoven fabric sheet was further laminated with a foamed polypropylene board with a thickness of 3 mm using a press-forming machine at 130°C for two minutes to obtain a laminated board.
The resulting laminated board was cut into a 10 cm square and put into a desiccator with an internal volume of 2 1 in which the atmosphere was adjusted to a formaldehyde concentration of 30 ppm. The formaldehyde concentration in the desiccator was measured after two hours. As a result, the formaldehyde concentration was 10 ppm.

### Example 14

A paint was prepared by adding 4 parts by mass of ammonium hydrogen phosphate with an average particle diameter of 5 µm (manufactured by Wako Pure Chemical Industries, Ltd.) to 100 parts by mass of an acrylic resin paint ("Aqueous Fresh 21" manufactured by Dai Nippon Toryo Co., Ltd., a resin content: 20 mass%) and mixing the ingredients.
The paint was sprayed to a board of A4 size plywood board with a thickness of 10 mm.
A 4 cm × 4 cm square specimen was cut from the plywood board and put into a 500 ml broad-mouth bottle. The bottle was covered. After allowing the bottle to stand at 60°C for five minutes, the formaldehyde concentration in the bottle was measured using a Kitagawa gas detector tube. As a result, the formaldehyde concentration was 0.5 ppm or less.

### Example 15

An adhesive composition was prepared by adding 10 parts by mass of ammonium polyphosphate (content of water-insoluble matter at 20°C: 90%) with an average particle diameter of 20 µm to 100 parts by mass of an acrylic resin adhesive ("Y650" manufactured by Cemedine Co., Ltd., resin content: 40 mass%) and mixing the ingredients.
A sheet of A4 size regular paper was bonded to a plywood board with a thickness of 10 mm using this adhesive.
A 4 cm × 4 cm square specimen was cut from the plywood board and put into a 500 ml broad-mouth bottle. The bottle was covered. After allowing the bottle to stand at 60°C for five minutes, the formaldehyde concentration in the bottle was measured using a Kitagawa gas detector tube. As a result, the formaldehyde concentration was 0.5 ppm or less.

### Comparative Example 11

An adhesive composition was prepared by adding 0.5 part by mass of ammonium polyphosphate with an average particle diameter of 20 µm to 100 parts by mass of an acrylic resin adhesive ("Y650" manufactured by Cemedine Co. , Ltd., a resin content: 40 mass%) and mixing the ingredients.
A sheet of A4 size regular paper was bonded to a plywood board with a thickness of 10 mm using this adhesive.
A 4 cm × 4 cm square specimen was cut from the plywood board and put into a 500 ml broad-mouth bottle. The bottle was covered. After allowing the bottle to stand at 60°C for five minutes, the formaldehyde concentration in the bottle was measured using Kitagawa gas detector tube. As a result, the formaldehyde concentration was 2 ppm.

### Example 16

The same experiment and evaluation as in Example 7 were conducted except for using ammonium polyphosphate with an average particle diameter of 6 µm (manufactured by Taihei Chemical Industrial Co., Ltd.) instead of the ammonium dihydrogen phosphate.
As a result, the formaldehyde concentration in the bottle was found to be 0.5 ppm.

### Example 17

An adhesive was prepared and evaluated in the same manner as in Example 1 except for adding 5 parts by mass of classified ammonium dihydrogen phosphate with an average particle diameter of 15 µm instead of ammonium dihydrogen phosphate with an average particle diameter of 5 µm.
As a result, the formaldehyde concentration in the bottle was found to be 0.5 ppm.

### Industrial Applicability

Since the deodorant of the invention has excellent formaldehyde deodorization capability, the deodorant can be used suitably for products which may emit formaldehyde such as an adhesive and a building material.
In addition, since the deodorant products using the deodorant of the invention can greatly suppress the amount of formaldehyde emitted therefrom, these products are highly safe to human bodies. Furthermore, the deodorant has a function of deodorizing formaldehyde emitted from another product. Therefore, the deodorant can be used in a wide variety of products which are used in a human living environment.

## Claims

1. A deodorant comprising a powder of an amine salt of a phosphorus inorganic acid, the powder having an average particle diameter of 0.1 to 30 µm.

2. The deodorant according to claim 1 wherein the amine salt of a phosphorus inorganic acid is an ammonium salt.

3. A deodorant product comprising the deodorant of claim 1 or 2.

4. A deodorant adhesive comprising 1 to 100 parts by mass of the deodorant of claim 1 or 2 relative to 100 parts by mass of an adhesive.

5. A deodorant paint comprising 1 to 100 parts by mass of the deodorant of claim 1 or 2 relative to 100 parts by mass of a paint.

6. A deodorant foamed material comprising 1 to 100 parts by mass of the deodorant of claim 1 or 2 relative to 100 parts by mass of a foamable resin.

7. A deodorant fiber treating agent comprising 1 to 60 parts by mass of the deodorant of claim 1 or 2.

8. A fiber product processed by using the deodorant fiber treating agent of claim 7.

9. The fiber product according to claim 8 which is a fiber, a woven fabric or a nonwoven fabric.

10. A deodorant ink comprising 1 to 100 parts by mass of the deodorant of claim 1 or 2 relative to 100 parts by mass of a dry ink resin.

11. A deodorant paper comprising 0.001 to 100 parts by mass of the deodorant of claim 1 or 2 relative to 100 parts by mass of an absolutely dried pulp.

12. A deodorant gypsum board comprising a composition comprising 97 to 70 parts by mass of a gypsum and 3 to 30 parts by mass of the deodorant of claim 1 or 2.

13. A deodorant synthetic resin product comprising 1 to 100 parts by mass of the deodorant of claim 1 or 2 relative to 100 parts by mass of a synthetic resin.

14. A plywood board comprising the deodorant of claim 1 or 2 applied thereon.

15. A hot melt product comprising 1 to 30 parts by mass of the deodorant of claim 1 or 2 relative to 100 parts by mass of a hot melt agent.
